# EUROPEAN PATENT SPECIFICATION

(11) **EP 0 359 258 B1**
(45) Date of publication and mention of the grant of the patent: **27.04.1994**
(21) Application number: 89117030.0
(22) Date of filing: 14.09.1989
(51) Int. Cl.: A61K 31/66

(54) **The use of inositoltrisphosphate for the preparing of a medicament against bone disorders**
Verwendung von Inositoltrisphosphat zur Herstellung eines Arzneimittels gegen Knochenerkrankungen
Utilisation de trisphospahte d'inositol pour préparer un médicament contre les maladies de l'os

(30) Priority: 15.09.1988 SE 8803248
(43) Date of publication of application: 21.03.1990
(73) Proprietor: PERSTORP AB, 284 80 Perstorp (SE)
(72) Inventor: Sirén, Matti, CH-6926 Montagnola Lugano (CH)
(74) Representative: Hansen, Bernd, Dr. Dipl.-Chem.

(56) References cited:
- CHEMICAL ABSTRACTS, vol. 33, no. 18, 20th September 1939, column 7351, abstract no. 73514 , Columbus, Ohio, US; M. GEDROYC et al.: "The antirachitic properties of some inorganic and organic compounds of phosphorus, particularly the inositolphosphates"
- J. DENT. RES., vol. 63, no. 6, 1984, pages 890-893; B.C. GOMES et al.: "Inhibitory effect of inositol phosphates on parathyroid hormone-induced bone resorption in organ cultures"
- J. ORAL PATHOL., vol. 15, no. 1, June 1986, pages 54-58; B.C. GOMES et al.: "Histologic study of the inhibition of bone resorption in organ cultures by myoinositol-2-monophosphate"

## Description

The present invention relates to the use of at least the specific isomer D-myo-inositol-1,2,6-trisphosphate (IP₃) for the preparing of a medicament for preventing, alleviating or combatting bone disorders in mammals including man.

Bone has many essential functions such as providing rigid support to extremities and to the body cavities that contain vital organs. Furthermore the bones are crucial to locomotion and provide a large reservoir of ions such as calcium and phosphorus.
Continuous processes occuring at bone surfaces are maintaining the integrity of the bone.
Abnormalities of these remodeling processes results in metabolic bone diseases and bone disorders.
Disorders are often due to changes in the status of vitamin D and its derivatives, levels of calcium, phosphorus, magnesium alkaline phosphatase and hormones from the parathyroid gland and the pituitary. These diseases have a relatively higher incidence in e.g. the aging population and for women after the menopause.
Osteomalacia and rickets are examples of abnormal mineralization in general of bone while e.g. hypercalcemia and hyperparathyroidism are disorders related to more specific parameters. Osteoporosis is a disease where the absolute amount of bone decreases below the level required for mechanical support. Different forms of osteitis such as Paget's disease are also related to bone disorders.
In the effort of treating diseases related to bone disorders different therapeutic agents are used but with limited success. Vitamin D or derivatives thereof, supplementation of calcium or treatment with calcitonin are examples of drugs used in this area. For the treatment of osteoporosis also estrogen is used while fluoride also is used for the treatment of Paget's disease.
The above mentioned drugs only regulate symptoms of different conditions but do not effect the underlying factors of the diseases. Furthermore many of the used drugs give severe side effects or have other inconvenient properties for the patients. For example the use of estrogen increases the incidence of breast cancer as well as thrombosis. Calcitonin must be administered parenterally and some patients develop resistance to its biological effects and allergic reactions. Fluoride is quite toxic and gives rise to gastric irritations.
This implies a marked need for more effective and safe pharmacological compounds in this area.

According to the present invention it has surprisingly become possible to overcome and reduce the above mentioned problems by the use of at least the specific isomer D-myo-inositol-1,2,6-trisphosphate (IP₃) for the preparing of a medicament for preventing, alleviating or combatting bone disorders in mammals including man, wherein the bone disorders are related to bone demineralization, osteoporosis, Paget's disease, bone erosion and periostitis.

From the European Patent No. 179439 a pharmaceutical composition comprising as a pharmaceutically active ingredient at least one isomer of inositoltrisphosphate is known. In said patent the effect of this pharmaceutical composition is shown for different areas such as cardiovascular diseases.

J. Dent. Res. 63 (1984), 890-893 investigates the properties of phytic acid and its inositolphosphate derivatives which can differ in the number of phosphate atoms on bone resorption. Inter alia IP₃ fractions are also mentioned which can be obtained by hydrolysis of phytate by phosphatase enzymes or by acid (first page, right column, first full paragraph). As is clear from page 890, last full paragraph, in the hydrolysis resulting in fractions of various isomers containing a certain amount of phosphate atoms, "no attempt was made to separate isomers in this study", so that the conclusions made are based on the behaviour of a fraction, which may contain a whole series of many structural isomers. The separation and identification of specific isomers being very difficult.

In the said reference background art is also mentioned wondering about the in vitro effects of IP's and diphosphonates on bone and bone cells and that it has to be ultimately determined whether undesirable effects similar to those resulting from the use of diphosphonates are found when phytate or its derivatives are used systematically in the treatment of metabolic bone diseases.

The present invention relates to different bone disorders such as: osteoporosis, Paget's disease, erosion of bones, e.g. destruction of articular surfaces, and different forms of periostitis.

In preferred embodiments of the invention the bone disorders relate to osteoporosis, erosion of bones, periostitis and Paget's disease.

The production of IP₃ and the isolation of the different isomers thereof are disclosed in the US patent No 4.777.134. The IP₃ isomers can also be produced by synthetic methods, chemically or enzymatically, starting with e.g. inositol and a phosphorus source. Furthermore, microbiological production methods including hybrid DNA-techniques of IP₃ are also suitable.

The structure of IP₃ and the different isomers thereof are disclosed in the US patent No 4.735.936 and the US patent No 4.797.390.

It is suitable that the medicament used according to the invention exists in unit dosage form. Tablets, granules or capsules are suitable administration forms for such unit dosage. Furthermore, tablets and granules can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the medicament. The tablets or granules can also contain a disintegrant which causes the tablets or the granules, respectively, to disintegrate easily in the intestine. In certain cases, especially in acute situations, it is preferable to use the unit dosage in the form of a solution for intravenous administration.

The medicament can also consist as such of IP₃ solely without any additive, excipient or carrier.

If desired, the medicament can be free of other inositol phosphates, IP₁, IP₂, IP₄, IP₅ and IP₆. Accordingly, the mixture of IP₃ isomers can have a purity of 90-100%, such as 93-100% or preferably 95-100%.

Alternatively, the medicament can consist of or comprise one or more specific IP₃ isomers, each present in substantially pure form. Thus, the different isomers can be isolated from each other in substantially pure form, which means that they have a purity of 80-100%, such as 82-100% or 85-100%, preferably 90-100%. Since the isomers can be produced in pure form they can be mixed in any proportion, of course.

The medicament can consist of IP₃, wherein said IP₃ is provided by at least one of IP₆, IP₅ or IP₄ and a degradative substance such as an enzyme suitable to form IP₃.

It is in most cases suitable that the IP₃-isomer or isomers used for the preparing of the medicament according to the invention is present in salt form in order not to affect the mineral balance negatively. The salt should preferably consist of a sodium, potassium, calcium, zinc or magnesium salt or a mixture of two or more of these salts. Calcium and zinc salts or mixtures of these are especially preferred. The isomer of IP₃ can also partly be present as a salt of one or more physiologically acceptable compounds in the lanthanide series.

For the above mentioned reasons it is also an advantage if the medicament contains a surplus or an extra addition of at least one pharmaceutically acceptable salt of calcium, zinc or magnesium with a mineral acid or organic acid. This is especially valuable for elderly persons who are often deficient in these minerals.

For administration to human patients appropriate dosages can routinely be determined by those skilled in this art by extension of the results obtained in animals at various dosages. The preferred dosage for humans falls within the range of 0.1 to 1000 mg, especially 0.1 to 200 mg IP₃/day/kg body weight.

In animal experiments, no toxic effects were seen after administration of very high doses of IP₃, 160 mg/kg body weight by intraperitoneal injection to mice.

The medicament usually contains 0.01-1.5 g, such as 0.05-1.3 or preferably 0.1-1 g of IP₃ per unit dosage.

According to the invention the isomer of IP₃ is D-myo-inositol-1,2,6-trisphosphate.

One type of function of the medicament is to reverse, prevent or alleviate damage to membranes of different cell types, but especially cell membranes of platelets, erythrocytes and endothelial cells. The use of the medicament results in an improved stabilization, a decreased susceptibility to deformation and an improved function of the cell types. Furthermore, parameters such as aggregability and adhesion to e.g. vessel walls are decreased.
The use of the medicament affects the levels and the activities of alkaline phosphatase.
Furthermore a normalization of the inositol metabolism is measured when the medicament is used. Other results of the use of the medicament are regulation of membrane fluidity, the incorporation of components such as cholesterol and the production, incorporation and balance between different phospholipids.

Another result of the use of the medicament is the regulation of the electrolyte balance of the cell. As examples, regulation of intracellular and extracellular levels of calcium, potassium, chloride, phosphate etc. can be mentioned.

The cell surface activity and responsiveness to external stimuli via receptor activation /deactivation due to for example phosphorylation are other parameters effected by the use of the medicament.

The invention will be further explained with the following examples. Examples 1-2 show the production of IP₃ whereas examples 3 and 4 illustrate the preparation of solutions and tablets of D-myo-inositol-1,2,6-trisphosphate. Examples 5-7 relate to the effect of IP₃ on different types of bone disorders.

### Example 1

Hydrolysis of sodium phytate with baker's yeast and fractionation of a mixture of inositol phosphates.

A 1.4 kg quantity of sodium phytate (from corn, Sigma Chemical Co) was dissolved in 1 200 1 sodium acetate buffer pH 4.6. 100 kg of baker's yeast from Jästbolaget, Sweden (dry substance: 28 %, nitrogen content: 2 %, phosphorus content: 0.4 %) was added with stirring and incubation was continued at 45^{o}C. The dephosphorylation was followed by determining the inorganic phosphorus released. After 7 hours when 50 % inorganic phosphorus was liberated the hydrolysis was stopped by adding ammonia to pH 12. The suspension was centrifuged and the supernatant was collected.

800 1 of the supernatant was passed through an ion-exchange column (Dowex 1, chloride form, 100 cm x 150 cm) and eluted with a linear gradient of hydrochloric acid (0-0.7 N HCL).

### Example 2

Structural determination of isomers of inositoltrisphosphate.

The fraction obtained in example 1 with a phosphorus/ inositol ratio of three to one was neutralized and precipitated as a calcium salt. 100 mg of the precipitate was analyzed with H-NMR. Data show that the peak consists of D-myo-inositol-1,2,6-trisphosphate.

### Example 3

Solution of sodium salt of D-myo-inositol-1,2,6-trisphosphate for injection.

0.5 g of the sodium salt of IP₃ and 0.77 g NaCl were dissolved in 98.73 ml of water for injection to form a solution suitable for injection into a person or an animal.

### Example 4

Tablets of calcium salt of D-myo-inositol-1,2,6-trisphosphate.

Tablets of the calcium salt of D-myo-inositol-1,2,6-trisphosphate were produced in the following way. 50 g calcium salt of D-myo-inositol-1,2,6-trisphosphate, 132 g lactose and 6 g acacia were mixed. Purified water was then added to the mixture, whereupon the mixing was continued until a suitable consistency was obtained. The mixture was sieved and dried. Then the mixture was blended with 10 g talcum and 2 g magnesium stearate. The mixture was compressed into tablets each weighing 200 mg.

### Example 5

Injection of Freund's complete adjuvant into rats results in the development of different forms of bone disorders.
13 male rats weighing approximately 200 grams were divided into two groups.
The animals in both groups were anaesthetized with halothane and injected intradermally at the tail base with 0.1 ml adjuvant solution (Myo-bacterium butylricum, 10 mg/ml in paraffin oil) at day 0.
The seven rats in Group 1 received daily subcutaneous injections of 50 mg D-myo-inositol-1,2,6-trisphosphate (IP₃) during the experiment (25 days) while the six rats in Group 2 served as a control and received daily injections with saline. At the end of the experiment the animals were killed and the left hind limbs were fixed in a saline-solution, washed in water, dried and x-rayed. The x-ray unit is of the microfocal type and the produced radiographs have a magnification of x 10.
Each radiograph was graded blindly for the presence and severity of bone mineralization with the following grades:
- Grade 0:: Normal
- Grade 1:: Mild articular osteoporosis
- Grade 2:: Severe generalized osteoporosis with pathological fractures

The results are shown in the following table:

| | | | | | | | |
|---|---|---|---|---|---|---|---|
| Animals no. | 1 | 2 | 3 | 4 | 5 | 6 | 7 (grade) |
| Group 1 | 0 | 1 | 1 | 1 | 0 | 1 | 1 |
| Group 2 | 1 | 2 | 1 | 1 | 0 | 2 | - |

As can be seen the IP₃-treatment (Group 1) reduces the occurence and severity of osteoporosis compared to control (Group 2).

### Example 6

The x-rays obtained as described in example 5 were graded blindly for the presence and severity of bone erosions with the following grades:
- Grade 0:: Normal
- Grade 1:: Small bony irregulations at corners of articular surfaces
- Grade 2:: Complete destruction of the articular surfaces

The results are shown in the following table:

| | | | | | | | |
|---|---|---|---|---|---|---|---|
| Animal no. | 1 | 2 | 3 | 4 | 5 | 6 | 7 (grade) |
| Group 1 | 0 | 1 | 1 | 0 | 1 | 0 | 0 |
| Group 2 | 1 | 2 | 2 | 1 | 1 | 2 | - |

The IP₃-treatment (Group 1) reduces the occurence and severity of bone erosion compared to control (Group 2).

### Example 7

The x-rays obtained as described in example 5 were graded blindly for the presence and severity of periostitis with the following grades:
- Grade 0:: Normal
- Grade 1:: Thin layers of new bone
- Grade 2:: Severe irregular bony proliferation

The results are shown in the following table:

| | | | | | | | |
|---|---|---|---|---|---|---|---|
| Animal no. | 1 | 2 | 3 | 4 | 5 | 6 | 7 (grade) |
| Group 1 | 1 | 1 | 1 | 1 | 0 | 0 | 0 |
| Group 2 | 0 | 2 | 2 | 1 | 1 | 1 | - |

The IP₃-treatment (Group 1) reduces the occurence and severity of periostitis compared to control (Group 2).

## Claims

1. The use of at least the specific isomer D-myo-inositol-1,2,6-trisphosphate for the preparing of a medicament for preventing, alleviating or combatting bone disorders in mammals including man wherein the bone disorders are related to bone demineralization, osteoporosis, Paget's disease, bone erosion and periostitis.

2. The use according to claim 1, wherein said inositoltrisphosphate is in salt form.

3. The use according to claim 2, wherein said inositoltrisphosphate salt is a salt of sodium, potassium, calcium or zinc.

4. The use according to any one of claims 1-3, wherein the medicament is in tablet or granulated form.

5. The use according to any one of claims 1-3, wherein the medicament is in the form of a solution.

## Patentansprüche

1. Verwendung von mindestens dem spezifischen Isomer D-myo-Inosit-1,2,6-trisphosphat zur Herstellung eines Medikaments zur Prävention, Linderung oder Bekämpfung krankhafter Knochenstörungen bei Säugetieren, einschließlich dem Mensch, wobei die Knochenerkrankungen Knochenentmineralisierung, Osteoporose, Paget's Krankheit, Knochenerosion und Periostitis betreffen.

2. Verwendung gemäß Anspurch 1, worin das genannte Inosittrisphosphat in Salzform vorliegt.

3. Verwendung gemäß Anspruch 2, worin das genannte Inosittrisphosphat-Salz ein Salz von Natrium, Kalium, Calcium oder Zink ist.

4. Verwendung gemäß jedem Anspruch 1 bis 3, worin das Medikament in Tabletten- oder Granulatform vorliegt.

5. Verwendung gemäß jedem Anspruch 1 bis 3, worin das Medikament in Form einer Lösung vorliegt.

## Revendications

1. Utilisation d'au moins l' isomère spécifique 1,2,6-trisphosphate de D-myo-inositol, pour la préparation d'un médicament pour prévenir, soulager ou combattre les troubles osseux chez les mammifères, y compris l'homme, ces troubles osseux étant liés à une déminéralisation de l'os, à une ostéoporose, à une maladie de Paget, à une érosion de l'os et à une périostite.

2. Utilisation selon la revendication 1, dans laquelle ce trisphosphate d'inositol est sous forme de sel.

3. Utilisation selon la revendication 2, dans laquelle ce sel du trisphosphate d'inositol est un sel de sodium, de potassium, de calcium ou de zinc.

4. Utilisation selon l'une quelconque des revendications 1 à 3, dans laquelle le médicament est sous forme de comprimés ou de granulés.

5. Utilisation selon l'une quelconque des revendications 1 à 3, dans laquelle le médicament est sous la forme d'une solution.
